(19) Europäisches Patentamt
European Patent Office
Office européen des brevets

(11) Veröffentlichungsnummer: **0 264 348 B1**

(12) **EUROPÄISCHE PATENTSCHRIFT**

(45) Veröffentlichungstag der Patentschrift: **15.12.93**

(51) Int. Cl.[5]: **C07D 239/42**, C07D 239/46, C07D 239/48, A01N 47/30

(21) Anmeldenummer: **87810580.8**

(22) Anmeldetag: **07.10.87**

(54) **Harnstoffe.**

(30) Priorität: **13.10.86 CH 4074/86**
**28.07.87 CH 2869/87**

(43) Veröffentlichungstag der Anmeldung:
**20.04.88 Patentblatt 88/16**

(45) Bekanntmachung des Hinweises auf die Patenterteilung:
**15.12.93 Patentblatt 93/50**

(84) Benannte Vertragsstaaten:
**CH DE GB LI**

(56) Entgegenhaltungen:
**EP-A- 0 135 472**
**EP-A- 0 172 786**

(73) Patentinhaber: **CIBA-GEIGY AG**
**Klybeckstrasse 141**
**CH-4002 Basel(CH)**

(72) Erfinder: **Rempfler, Hermann, Dr.**
**Brücklismattstrasse 16**
**CH-4107 Ettingen(CH)**
Erfinder: **Stamm, Erich, Dr.**
**Mühle Sonnegg**
**CH-4950 Huttwil(CH)**
Erfinder: **Thummel, Rudolph C.**
**Le Borbet**
**CH-2892 Courgenay(CH)**

**EP 0 264 348 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-(2-Nitrophenyl)-N-pyrimidin-2-yl-harnstoffe mit herbizider und pflanzenwuchsregulierender Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses sowie Verfahren zur Herstellung der neuen Verbindungen. Ferner betrifft die Erfindung auch neue Zwischenprodukte und Verfahren zu deren Herstellung.

Aus der Patentschrift DD-151 404 und der europäischen Patentanmeldung EP-A-O 172 786 sind (Pyrimidin-2-yl)-2-nitroaniline bekannt geworden. Diese Verbindungen sind fungizid wirksam. Demgegenüber wurde überraschenderweise gefunden, dass N-Pyrimidin-2-yl-N-2-nitrophenylharnstoffe herbizide bzw. pflanzenwachstumsregulatorische Wirkung haben.

Die Erfindung betrifft somit Harnstoffe der Formel

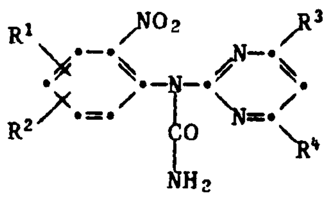

(I),

worin

$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyano,

$R^3$ und $R^4$ unabhängig voneinander Wasserstoff, Halogen, Cyano, OH, SH, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-Alkylamino, $C_1$-$C_4$-Alkoxycarbonyl oder $C_1$-$C_4$-Alkoxy-($C_1$-$C_4$)-alkyl bedeutet, sowie deren Salze und Additionsverbindungen mit Säuren, Basen und Komplexbildnern.

Im Rahmen der hier offenbarten Erfindung umfassen die angegebenen generischen Begriffe beispielsweise die folgenden spezifischen Einzelsubstituenten, wobei diese Aufzählung keine Einschränkung der Erfindung darstellt:

Alkyl umfasst die geradkettigen oder verzweigten $C_1$-$C_6$-Alkyle, wie Methyl, Ethyl, n-Propyl, iso-Propyl, n-Butyl, sek-Butyl, tert-Butyl, iso-Butyl, die isomeren Pentyle- wie etwa tert-Pentyl (1,1-Dimethylpropyl), iso-Pentyl (1-Ethylpropyl), sowie die isomeren Hexylreste. Bevorzugt sind die $C_1$-$C_3$-Alkylreste.

Halogen ist Fluor, Chlor, Brom und Jod. Bevorzugt ist Fluor, Chlor und Brom.

Mit Halogenalkyl sind die ganz oder teilweise gleich oder unterschiedlich halogensubstituierten Alkyle, gemäss der jeweiligen Definitionsbreite gemeint, wie etwa 2,2,2-Trifluorethyl, 1,1,1,3,3,3-Hexafluorprop-2-yl, Trifluormethyl oder Difluormethyl.

Als $C_1$-$C_4$-Alkoxycarbonylreste sind unter anderem Methoxycarbonyl, Ethoxycarbonyl sowie die isomeren Propyloxycarbonyle und Butyloxycarbonyle zu nennen.

Alkoxy sind im Rahmen der jeweiligen Definitionsbreite der isomeren Alkyloxyradikale, in erster Linie sind Methoxy und Ethoxy gemeint.

Halogenalkoxy und Halogenalkylthio sind im Rahmen der jeweiligen Definitionsbreite die isomeren ein- oder mehrfach gleich oder verschieden halogensubstituierten Alkylreste, wie etwa Trifluormethoxy, 2,2,2-Trifluorethoxy, Trifluormethylthio, 2,2,3,3,3-Pentafluorpropyloxy oder 1,1,2,2-Tetrafluorethoxy.

Als Alkoxyalkylreste sind unter anderem zu nennen: 2-Ethoxyethyl, 2-Methoxyethyl, 3-Methoxypropyl, 2-Methoxy-1-methylethyl und Methoxymethyl.

Di-$C_1$-$C_4$-Alkylamino ist eine mit gleichen oder verschiedenen Alkylresten substituierte Gruppe, wie Dimethylamino, Diethylamino, Methylethylamino.

In den weiteren Substituenten, welche aus mehreren Grundelementen zusammengesetzt sind, können die Teilelemente innerhalb der Definitionsbreite frei gewählt werden und obige Bedeutung haben.

Hervorzuheben sind Verbindungen der Formel I, worin

$R^1$ und $R^2$ unabhängig voneinander Wasserstoff, Halogen, $C_1$-$C_3$-Alkyl, $C_1$-$C_3$-Halogenalkyl, $C_1$-$C_3$-Alkoxy, $C_1$-$C_3$-Alkoxycarbonyl, Nitro, Cyano oder $C_1$-$C_3$-Alkoxy-($C_1$-$C_3$)-alkyl,

$R^3$ und $R^4$ unabhängig voneinander Wasserstoff, Halogen, $C_1$-$C_3$-Alkyl, $C_1$-$C_3$-Halogenalkyl, $C_1$-$C_4$-Alkoxy, $C_1$-$C_3$-Halogenalkoxy, $C_1$-$C_4$-Alkylthio, Amino, Mono-$C_1$-$C_3$-alkylamino, Di-$C_1$-$C_3$-alkylamino, Cyano, $C_1$-$C_4$-Alkoxycarbonyl oder $C_1$-$C_4$-Alkoxy-($C_1$-$C_4$)-alkyl

bedeutet.

Bevorzugt sind solche Verbindungen der Formel I, worin
$R^1$ Wasserstoff, Halogen, $C_1$-$C_3$-Alkyl, $C_1$-$C_3$-Halogenalkyl, $C_1$-$C_3$-Alkoxy oder $C_1$-$C_3$-Alkoxycarbonyl,
$R^2$ Wasserstoff, Halogen, $C_1$-$C_3$-Alkyl, Nitro oder Cyano,
$R^3$ und $R^4$ unabhängig voneinander Wasserstoff, Halogen, $C_1$-$C_3$-Alkyl, $C_1$-$C_3$-Halogenalkyl, $C_1$-$C_4$-Alkoxy, $C_1$-$C_3$-Halogenalkoxy, $C_1$-$C_4$-Alkylthio, Amino, Mono-$C_1$-$C_3$-alkylamino, Di-$C_1$-$C_3$-Alkylamino, Cyano, $C_1$-$C_4$-Alkoxycarbonyl oder $C_1$-$C_3$-Alkoxy-($C_1$-$C_3$)-alkyl
bedeutet.

Insbesondere betrifft die Erfindung Verbindungen der Formel I, worin
$R^1$ Wasserstoff, Chlor, Methyl, Trifluormethyl, Methoxy oder Ethoxycarbonyl,
$R^2$ Wasserstoff, Chlor, Methyl oder Nitro,
$R^3$ Wasserstoff, Fluor, Chlor, Methyl, Ethyl, Propyl, Trifluormethyl, Methoxy, Ethoxy, Difluromethoxy, 1,1,2,2-Tetrafluorethoxy, Dimethylamino, Ethoxycarbonyl oder $C_1$-$C_4$-Alkylthio,
$R^4$ Wasserstoff, Fluor, Chlor, Brom, Methyl, Methoxymethyl, Methoxyethyl, $C_1$-$C_4$-Alkoxy, 1,1,2,2-Tetrafluorethoxy, Dimethylamino, Difluormethoxy, Fluormethoxy, Trifluormethyl oder $C_1$-$C_4$-Alkylthio
bedeutet.

Als besonders bevorzugt sind Verbindungen der Formel I zu nennen, worin
$R^1$ Wasserstoff, Methyl, Methoxy, Trifluormethyl oder Cl,
$R^2$ Wasserstoff oder Methyl,
$R^3$ Methyl, Ethyl, Propyl, Chlor, Trifluormethyl, 1,1,2,2-Tetrafluorethoxy, Methylthio, Propylthio, Ethoxy oder Methoxy,
$R^4$ Fluor, Chlor, Methyl, Methoxy, Methoxymethyl, Difluormethoxy, Fluormethoxy, Trifluormethyl oder Brom
bedeutet.

Hinsichtlich der Position der Substituenten $R^1$ und $R^2$ sind solche Verbindungen der Formel I bevorzugt, in welcher einer oder beide Substituenten in Position 5 bzw. 6 des Phenylrings gebunden sind.

Weiterhin sind besonders bevorzugt Verbindungen der Formel I, worin $R^1$ und $R^2$ Wasserstoff, $R^3$-Methoxy oder Methyl und $R^4$ Chlor, Methyl, Methoxy, Methoxymethyl, Trifluormethyl oder Difluormethoxy bedeutet.

Die Verbindungen der Formel I können hergestellt werden, dadurch dass man

a) ein Anilin der Formel II mit Phosgen zu einem Carbaminchlorid der Formel III umsetzt und dieses in einer zweiten Stufe mit $NH_3$ zu

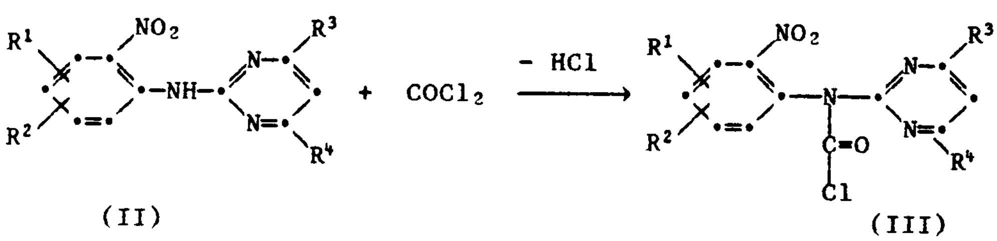


(II) (III)

$$III + NH_3 \xrightarrow{-\ HCl} I$$

einem Harnstoff der Formel I umsetzt oder

b) ein Anilin der Formel II mit Halogensulfonylisocyanat zu einem Halogensulfonylharnstoff der Formel IV umsetzt

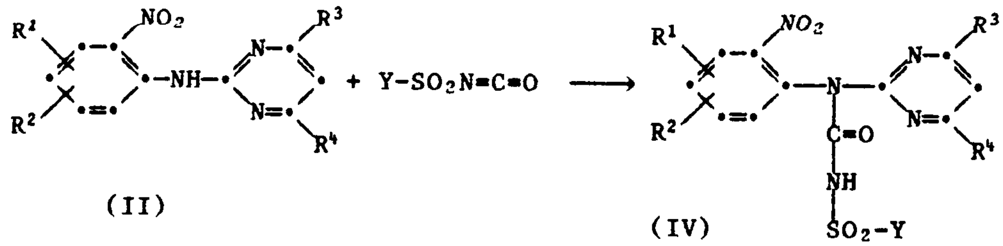


(II) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht oder
c) einen Sulfonylharnstoff der Formel V unter Einwirkung einer wässrigen Base zu einem Harnstoff der Formel I umlagert

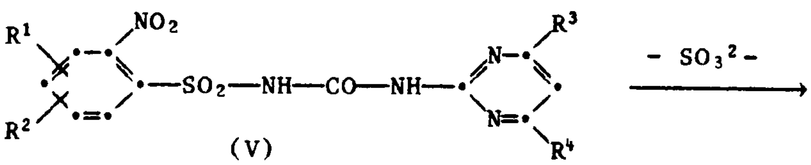


(V) (I),

wobei als wässrige Basen vorzugsweise NaOH/Wasser oder KOH/Wasser verwendet werden.

Die unter Halogenwasserstoffabspaltung bzw. HY-Eliminierung verlaufenden Umsetzungen II → III, III → I und IV → I werden vorzugsweise unter Verwendung säurebindender Mittel (Basen) durchgeführt.

Als solche kommen organische oder anorganische Basen in Betracht, z.B. tertiäre Amine wie Trialkylamine (Trimethylamin, Triethylamin, Tripropylamin usw.), Pyridine (Pyridin, 4-Dimethylaminopyridin, 4-Pyrrolidylaminopyridin usw.), Alkoholate wie z.B. Kalium-tert.-butylat, Natriummethylat oder -ethylat. Die zuvor genannten Reaktionen wie auch die Umsetzung V → I, können auch unter Phasentransferbedingungen mit Basen nach an sich bekannten Verfahren durchgeführt werden (Lit. Dehmlow & Dehmlow, Phase Transfer Catalysis Verlag Chemie, Weinheim, 1983).

Bei den Verfahrensvarianten a) und b) können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphstische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Einige der 2-Nitroaniline der Formel II sind Aus der EP-A 172 786 bekannt. Sie sind ebenso wie die neuen Carbamoylchloride der Formel III und die neuen Harnstoffe der Formel IV wertvolle Zwischenverbindungen für die Synthese der herbizid wirksamen Harnstoffe I.

Die Erfindung betrifft somit auch die neuen Verbindungen der Formel

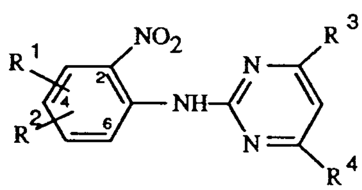

(II),

worin $R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyan, $R^3$ und $R^4$ unabhängig voneinander Wasserstoff, Halogen, Cyano, OH, SH, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-alkylamino, $C_1$-$C_4$-Alkoxycarbonyl oder $C_1$-$C_4$-Alkoxy-($C_1$-$C_4$)-alkyl bedeuten,

mit der Massgabe, dass $R^1$ nicht in 6-Stellung und $R^2$ nicht in 4-Stellung gebunden ist, wenn $R^1$ und $R^2$ für Wasserstoff, $NO_2$ oder $CF_3$ stehen, wobei nur $R^1$ oder $R^2$ für $NO_2$ stehen kann, und $R^3$ und $R^4$ Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Alkoxy($C_1$-$C_4$)alkyl ist, und $R^1$ oder $R^2$ nicht in 3-Stellung Halogen sein darf, wenn $R^2$ oder $R^1$ in 4-oder 6-Stellung Wasserstoff, $NO_2$ oder $CF_3$ ist und $R^3$ und $R^4$ Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$Alkoxy-($C_1$-$C_4$)alkyl ist.

Des weiteren betrifft die Erfindung Verbindungen der Formel

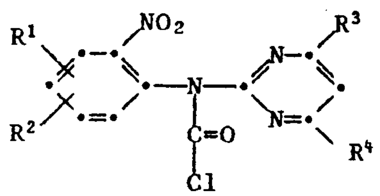

(III),

worin

$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$c_4$-Alkoxycarbonyl, $C_1$-$C_4$-Alkoxy-($C_1$-$C_4$)-alkyl, Nitro oder Cyano,

$R^3$ und $R^4$ unabhängig voneinander Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-alkylamino, $C_1$-$C_4$-Alkoxycarbonyl oder $C_1$-$C_4$-Alkoxy-($C_1$-$C_4$)-alkyl

bedeutet,

und Harnstoffe der Formel

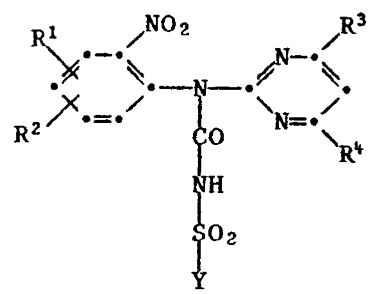

(IV),

worin

$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyano,

$R^3$ und $R^4$ unabhängig voneinander Wasserstoff, Halogen, Cyano, $C_1$-$C_6$-Alkyll, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-Alkylamino, $C_1$-$C_4$-Alkoxycarbonyl oder $C_1$-$C_4$-Alkoxy-($C_1$-$C_4$)-alkyl

Y Halogen, $C_1$-$C_4$-Alkoxy oder Phenoxy

bedeutet.

Die Verbindungen der Formel III und IV sind Zwischenprodukte der Verfahren a) und b) und können wie dort beschrieben aus den entsprechenden 2-Nitroanilinen der Formel II hergestellt werden.

Die neuen 2-Nitroaniline der Formel II sind analog literaturbekannter Verfahren herstellbar z.B. durch:

aa) Umsetzung von Guanidinen der Formel VI mit 1,3-Dicarbonylverbindungen der Formel VII

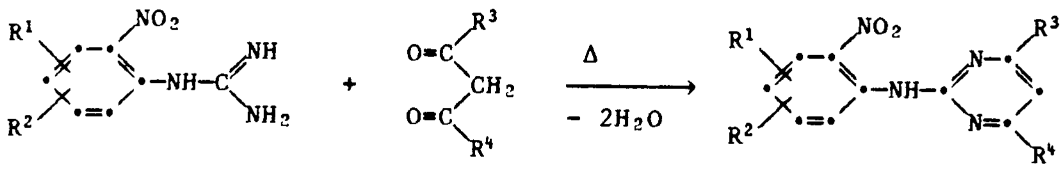


(VI) (VII) (II)

wobei die Kondensationsreaktion gewünschtenfalls in Gegenwart wasserbindender Mittel durchgeführt wird (Lit: D.J. Brown in "The Chemistry of Heterocyclic Compounds" Bd. VI 1962, Interscience Publ. New York) oder

bb) durch Umsetzung eines Halogenbenzols der Formel VIII mit einem 2-Aminopyrimidin der Formel IX

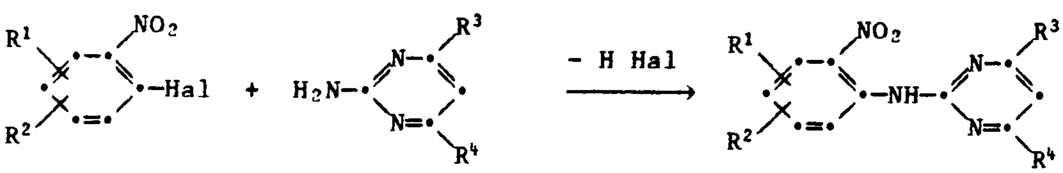


(VIII) (IX) (II)

wobei $R^1$ und $R^2$ vorzugsweise in 4 bzw. 6-Stellung des Phenylsystems (in II) gebunden sind und für eine elektronenanziehende Gruppe stehen (Lit. EP-A-0 172 786).

oder

cc) durch Abbau eines Sulfonylharnstoffes der Formel V unter Einwirkung einer wässrigen Base

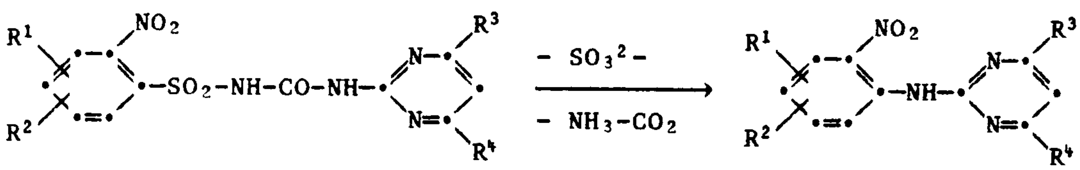


(V) (II)

bei erhöhter Temperatur.

Des weiteren betrifft die Erfindung herbizide und pflanzenwuchsregulatorische Mittel enthaltend eine Verbindung der Formel I zusammen mit geeigneten Hilfs- und/oder Trägerstoffen.

Die Wirkstoffe der Formel I werden in der Regel mit Aufwandmengen von 0,005 bis 5 kg/ha insbesondere 0,1 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Mais und Reis befähigen.

Die Verbindungen der Formel I haben ausserdem pflanzenwuchsregulatorische Eigenschaften. Es werden sowohl Monokotyledonen als auch Dikotyledonen in ihrem Wachstum beeinflusst.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumsregulatoren beruht darauf, dass die Nährstoffe in stärkerem Masse der Blüten- und Fruchtbildung zugute kommen, während das vegetative

Wachstum eingeschränkt wird.

Bei grösseren Aufwandmengen werden Unkräuter und Gräser in ihrer Entwicklung so geschädigt, dass sie absterben.

Die Erfindung betrifft auch herbizide und pflanzenwachstumsregulierende Mittel, welche einen Wirkstoff der Formel I enthalten, sowie Verfahren zur pre- und post-emergenten Unkrautbekämpfung und zur Beinflussung des Pflanzenwuchses von monokotylen und dikotylen Pflanzen, insbesondere Gräsern, tropischen Bodenbedeckern und Geiztrieben.

Die Verbindungen der Formel I werden in unveränderter Form oder vorzugsweise als Mittel zusammen mit den in der Formulierungstechnik üblichen Hilfsmitteln eingestzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalzezu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.

Dr. Helmut Stache "Tensid Taschenbuch"
Carl Hanser Verlag, München/Wien 1981.

Die Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

| Emulgierbare Konzentrate: | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

| Stäube: | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

| Suspensions-Konzentrate: | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

| Benetzbare Pulver: | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

| Granulate: | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Die - unkorrigierten - Schmelzpunkte in den nachfolgenden Herstellungsbeispielen sind in °C angegeben.

H 1.1. Synthese von N-(6-Chlor-4-methylpyrimidin-2-yl)-N-2-nitrophenyl-harnstoff

79.3 g (0.3 Mol) N-(6-Chlor-4-methyl-pyrimidin-2-yl)-2-nitro-anilin werden bei 60° in 2.2 l Essigsäureethylester gelöst. Die Lösung wird auf 5° gekühlt, wobei gelbe Kristalle ausfallen. Dazu werden 56.6 g (0.4 Mol) Chlorsulfonylisocyanat gegeben und dann 15 min bei 5° gerührt. Dann werden 300 ml kaltes Wasser zugegossen, die organische Phase wird abgetrennt, mit Natriumsulfat getrocknet und eingedampft. Der feste Rückstand wird mit wenig Essigsäureäthylester verrieben.

Man isoliert 71.5 g (74.4 %) der Titelverbindung der Formel

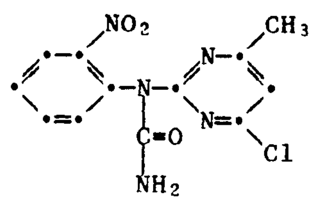

(Verbindung No. 1.007) als Kristalle vom Smp. 155°C).

H.1.2. Synthese von N-(6-Chlor-4-methyl-pyrimidin-2-yl)-N-2-nitrophenyl-harnstoff

18.6 g (0.05 Mol) 3-(6-Chlor-4-methyl-pyrimidin-2-yl)-1-(2-nitrophenylsulfonyl)-harnstoff werden in 100 ml Wasser und 200 ml Chloroform suspendiert. Dazu tropft man bei 25° innert 4 Std. eine Lösung von 3.2 g Natriumhydroxid in 350 ml Wasser. Die 2-phasige Lösung wird 16 Std. gerührt. Dann wird die $CHCl_3$-Phase abgetrennt, mit Natriumsulfat getrocknet und eingedampft. Der Rückstand wird aus Chloroform umkristallisiert.

Man isoliert 3,3 g (21.4 %) der Titelverbindung der Formel

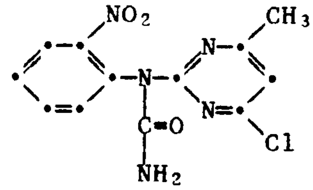

(Verbindung No. 1.007) als Kirstalle vom Smp. 155°C.

Analog zu den Herstellungsbeispielen H.1. können die nachstehend in Tabelle I aufgeführten Verbindungen der Formel

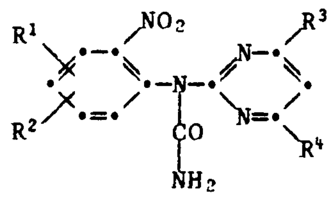

(I),

hergestellt werden:

Tabelle 1

| Verb. Nr. | $R^1$ | $R^2$ | $R^3$ | $R^4$ | physikalische Daten |
|---|---|---|---|---|---|
| 1.001 | H | H | $OCH_3$ | $OCH_3$ | Smp. 157-158° |
| 1.002 | H | H | $CH_3$ | $CH_3$ | ab 150° Zersetzung |
| 1.003 | H | H | $OCH_3$ | $CH_2OCH_3$ | Smp. 124-125° |
| 1.004 | H | H | $OCH_3$ | $CH_3$ | Smp. 153-154° |
| 1.005 | H | H | $CH_3$ | $OCHF_2$ | Smp. 144°, Zers. |
| 1.006 | H | H | $OCH_3$ | Cl | Smp. 172-175° |
| 1.007 | H | H | $CH_3$ | Cl | Smp. 155° |
| 1.008 | 4-Cl | H | $CH_3$ | $CH_3$ | Smp. 156°, Zers. |
| 1.009 | H | H | $CH_3$ | F | Smp. 134-135° |
| 1.010 | H | H | $CH_2CH_3$ | Cl | Smp. 136-137° |
| 1.011 | H | H | $CH_2CH_3$ | $OCHF_2$ | Smp. 132-134° |
| 1.012 | H | H | $CH_3$ | Br | Smp. 174-175° |
| 1.013 | H | H | Cl | Cl | Smp. 164-165° |
| 1.014 | H | H | F | F | |
| 1.015 | H | H | Cl | $OCHF_2$ | |
| 1.016 | H | H | $OCHF_2$ | $OCHF_2$ | |
| 1.017 | H | H | $OCH_2CH_3$ | $CH_3$ | Smp. 142-144° |
| 1.018 | H | H | $SCH_3$ | $CH_3$ | Smp. 180-182° |
| 1.019 | H | H | $SCH(CH_3)_2$ | $CH_3$ | Smp. 151-152° |
| 1.020 | H | H | $SCH_3$ | Cl | Smp. 133-135° |
| 1.021 | H | H | $SC_4H_9(n)$ | Cl | |
| 1.022 | H | H | COOEt | Cl | |
| 1.023 | H | H | COOEt | $CH_3$ | |
| 1.024 | H | H | $SCH_2CH_3$ | $OCHF_2$ | |
| 1.025 | H | H | $CF_3$ | Cl | ab 135° Zersetzung |
| 1.026 | H | H | $CF_3$ | F | |
| 1.027 | H | H | $CF_3$ | $OCH_2CH_3$ | |
| 1.028 | H | H | $CF_3$ | $SCH_3$ | |
| 1.029 | H | H | $CF_3$ | $N(CH_3)_2$ | |
| 1.030 | H | H | $N(CH_3)_2$ | Cl | |
| 1.031 | H | H | $N(CH_3)_2$ | $CH_3$ | |

Tabelle 1 (Fortsetzung)

| Verb. Nr. | $R^1$ | $R^2$ | $R^3$ | $R^4$ | physikalische Daten |
|---|---|---|---|---|---|
| 1.032 | H | H | $OCF_2CHF_2$ | $CH_3$ | Smp. 137-138° |
| 1.033 | H | H | $OCF_2CHF_2$ | $OCF_2CHF_2$ | |
| 1.034 | 4-$CH_3$ | H | $CH_3$ | Cl | Smp. 159-160° |
| 1.035 | 4-$CH_3$ | H | $CH_3$ | $OCHF_2$ | |
| 1.036 | 4-$CH_3$ | H | $CH_3$ | F | |
| 1.037 | 4-$CH_3$ | H | $CH_3$ | $OCF_2CHF_2$ | |
| 1.038 | 4-$CH_3$ | H | $CH_3$ | $SCH_3$ | |
| 1.039 | 4-$CF_3$ | H | $CH_3$ | Cl | |
| 1.040 | 4-$CF_3$ | H | Cl | Cl | |
| 1.041 | 4-$CF_3$ | H | $OCH_3$ | $CH_2OCH_3$ | |
| 1.042 | 4-$CF_3$ | H | $CH_3$ | H | |
| 1.043 | 4-$CF_3$ | H | H | H | |
| 1.044 | 4-Cl | H | $CH_3$ | Cl | Smp. 149-150° |
| 1.045 | 4-Cl | H | $CH_3$ | F | |
| 1.046 | 4-Cl | H | $CH_3$ | $OCHF_2$ | Smp. 128-129° |
| 1.047 | 4-Cl | H | $CH_3$ | $SCH_2CH_3$ | |
| 1.048 | 4-COOEt | H | $CH_3$ | $CH_3$ | |
| 1.049 | 4-COOEt | H | $CH_3$ | Cl | |
| 1.050 | 4-COOEt | H | $CH_3$ | $OCH_3$ | |
| 1.051 | H | 6-$CH_3$ | $CH_3$ | Cl | Smp. 160-161° |
| 1.052 | H | 6-$CH_3$ | Cl | Cl | |
| 1.053 | H | 6-$CH_3$ | Cl | $SCH_3$ | |
| 1.054 | 4-$OCH_3$ | H | $CH_3$ | Cl | |
| 1.055 | 4-$OCH_3$ | H | $CH_3$ | $CH_3$ | |
| 1.056 | 5-$CH_3$ | H | $CH_3$ | $CH_3$ | |
| 1.057 | 5-$CH_3$ | H | $CH_3$ | $OCHF_2$ | |
| 1.058 | 5-$CH_3$ | H | $CH_3$ | Cl | |
| 1.059 | 5-$CH_3$ | H | $CH_3$ | $OCF_2CHF_2$ | |
| 1.060 | 4-Cl | 6-Cl | $CH_3$ | $CH_3$ | |
| 1.061 | 4-Cl | 6-Cl | $CH_3$ | Cl | |
| 1.062 | 4-Cl | 6-Cl | Cl | Cl | |
| 1.063 | 4-Cl | 6-Cl | $CH_3$ | F | |
| 1.064 | 4-Cl | 6-Cl | Cl | $SC_2H_5$ | |
| 1.065 | 4-$CF_3$ | 6-$NO_2$ | $CH_3$ | Cl | |
| 1.066 | 4-$CF_3$ | 6-$NO_2$ | H | H | |
| 1.067 | 4-$CF_3$ | 6-$NO_2$ | $CH_3$ | H | |
| 1.068 | 4-$CF_3$ | 6-$NO_2$ | $CH_3$ | $OCHF_2$ | |
| 1.069 | 4-$CF_3$ | 6-$NO_2$ | $CH_3$ | $CH_2OCH_3$ | |
| 1.070 | 4-$CF_3$ | 6-$NO_2$ | $CH_3$ | $CH_3$ | |
| 1.071 | H | H | $C_3H_7(n)$ | Cl | Smp. 145-147° Zers. |
| 1.072 | H | H | $C_3H_7(n)$ | $OCH_2F$ | Smp. 117-118° |

Tabelle 1 (Fortsetzung)

| Verb. Nr. | $R^1$ | $R^2$ | $R^3$ | $R^4$ | physikalische Daten |
|---|---|---|---|---|---|
| 1.073 | H | H | $CF_3$ | Br | Smp. 154-155° |
| 1.074 | 4-Cl | H | $CF_3$ | Cl | Smp. 135-138° |
| 1.075 | 4-Cl | H | $CF_3$ | $OCHF_2$ | Smp. 145-150° |
| 1.076 | 4-$CF_3$ | H | $CH_3$ | $OCH_3$ | Smp. 159-160° |
| 1.077 | H | H | $CH_3$ | H | Smp. 143-144° |
| 1.078 | 4-$OCH_3$ | H | $CH_3$ | Cl | Smp. 164-165° |
| 1.079 | 4-Cl | H | $CH_3$ | Br | Smp. 180-182° |
| 1.080 | H | H | $CH_3$ | $CF_3$ | Smp. 140-141° |
| 1.081 | 5-Cl | H | $CH_3$ | Cl | Smp. 147-148° |
| 1.082 | H | 6-$CH_3$ | $CH_3$ | $CF_3$ | Smp. 174-175° |
| 1.083 | H | H | $CF_3$ | $CF_3$ | Smp. 168-171° |
| 1.084 | 5-Cl | H | $CH_3$ | $CF_3$ | |
| 1.085 | H | H | $CF_3$ | $OCH_3$ | Smp. 156-158° |
| 1.086 | H | H | $CF_3$ | $OC_4H_9(n)$ | Smp. 106-107° |
| 1.087 | H | H | $CF_3$ | $SCH(CH_3)_2$ | |
| 1.088 | H | H | $CF_3$ | $SC_2H_5$ | |
| 1.089 | H | H | $CF_3$ | $SC_4H_9(n)$ | |
| 1.090 | H | H | $CF_3$ | $OCH_2CH_2OCH_3$ | |
| 1.091 | H | H | $CF_3$ | $OC_3H_7(n)$ | |
| 1.092 | H | H | $CF_3$ | $OCF_2CHF_2$ | |
| 1.093 | H | 6-$CH_3$ | $CF_3$ | $OCH_3$ | |
| 1.094 | H | 6-$CH_3$ | $CF_3$ | $OC_2H_5$ | |
| 1.095 | H | 6-$CH_3$ | $CF_3$ | $OC_3H_7(n)$ | |
| 1.096 | H | 6-$CH_3$ | $CF_3$ | $OC_4H_9(iso)$ | |
| 1.097 | H | 6-$CH_3$ | $CF_3$ | $SCH_3$ | |
| 1.098 | H | 6-$CH_3$ | $CF_3$ | $SCH(CH_3)_2$ | |
| 1.099 | H | 6-$CH_3$ | $CF_3$ | $SC_4H_9(n)$ | |
| 1.100 | H | 6-Cl | $CH_3$ | Cl | |
| 1.101 | H | 6-Cl | $CH_3$ | $CF_3$ | |
| 1.102 | H | 6-Cl | $CF_3$ | $OCH_3$ | |
| 1.103 | H | 6-Cl | $CF_3$ | $OC_2H_5$ | |
| 1.104 | H | 6-Cl | $CF_3$ | $SCH_3$ | |
| 1.105 | H | 6-Cl | $CF_3$ | $SC_2H_5$ | |

H. 2.1. Synthese von N-(6-Chlor-4-methyl-pyrimidin-2-yl)-2-nitroanilin

24 g (0.064 Mol) 3-(6-Chlor-4-methyl-pyrimidin-2-yl)-1-(2-nitro-phenylsulfonyl)-harnstoff werden in 150 ml Wasser und 200 ml Chloroform suspendiert. Nach der Zugabe von 25 ml 30%iger Natronlauge wird das Reaktionsgemisch 1 Std. bei 50-55° gerührt, die Chloroform-Phase abgetrennt und mit Natriumsulfat getrocknet. Nach dem Abdampfen des Chloroforms erhält man 14.7 g (86.7 % d.Th.) der Titelverbindung der Formel

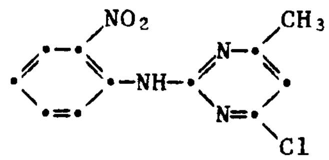

(Verbindung No. 2.007) als Kristalle vom Smp. 175 ° C.

H. 2.2. Synthese von N-(6-Chlor-4-methyl-pyrimidin-2-yl)-2-nitroanilin

Eine Mischung von 160 g (0.65 Mol) N-(6-Hydroxy-4-methyl-pyrimidin-2-yl)-o-nitroanilin, 2 ml N,N-Dimethylformamid und 120 ml Phosphoroxychlorid werden 3 Std. auf Rückfluss geheizt. Nach dem Abkühlen wird der schwarze Brei in Chloroform gelöst, diese Lösung zu 1 l Wasser zugetropft. Durch Zugabe von Eis wird die Temperatur bei 35 ° gehalten. Nach 1 Std. wird die organische Phase abgetrennt, die wässrige Phase mit Chloroform gewaschen. Beide Chloroformlösungen werden zusammen getrocknet und eingedampft. Der schwarze Rückstand wird in heissem Toluol gelöst, filtriert und durch Zugabe von Hexan zum Kristallisieren gebracht. Man erhält 104,9 g (61 %) der Titelverbindung der Formel

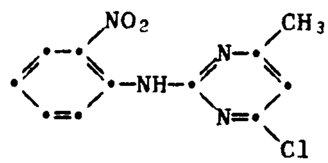

(Verbindung No. 2.007) als Kirstalle vom Smp. 175 ° C.

H. 2.3. Synthese von N-(6-Hydroxy-4-methyl-pyrimidin-2-yl)-2-nitroanilin

121 g (0.5 Mol) 2-Nitrophenyl-guanidin-carbonat, 100 g Acetessigsäureäthylester und 200 ml Diäthylenglykoldimethyläther werden zunächst auf 65 ° und nach Beendigung der $CO_2$-Entwicklung auf 140 ° erwärmt. Dabei lässt man Aethanol und Wasser abdestillieren. Nach 3 1/2 Std. lässt man die Suspension abkühlen, gibt Wasser zu und stellt den pH mit konzentrierter Salzsäure auf 4-5. Der gelbe Niederschlag wird abfiltriert, mit Wasser gewaschen und im Vakuum bei 80 ° C getrocknet. Man erhält 80.3 g (65.2 % d.Th.) der Titelverbindung der Formel

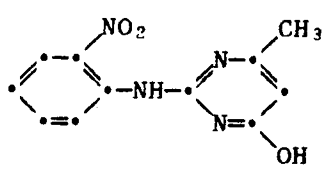

(Verbindung No. 2.071) als Kristalle vom Smp. 240-242 °.

Analog zu den Herstellungsbeispielen H.2. können die nachstehend in Tabelle 2 aufgeführten Verbindungen der Formel

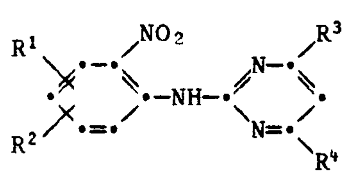

(II),

hergestellt werden:

Tabelle 2

| Verb. Nr. | $R^1$ | $R^2$ | $R^3$ | $R^4$ | physikalische Daten |
|---|---|---|---|---|---|
| 2.001 | H | H | $OCH_3$ | $OCH_3$ | Smp. 174-175° |
| 2.002 | H | H | $CH_3$ | $CH_3$ | Smp. 163-164° |
| 2.003 | H | H | $OCH_3$ | $CH_2OCH_3$ | Smp. 121-123° |
| 2.004 | H | H | $OCH_3$ | $CH_3$ | Smp. 144-145° |
| 2.005 | H | H | $CH_3$ | $OCHF_2$ | Smp. 104° |
| 2.006 | H | H | $OCH_3$ | Cl | Smp. 160° |
| 2.007 | H | H | $CH_3$ | Cl | Smp. 175° |
| 2.008 | H | H | $CH_3$ | F | Smp. 126-130° |
| 2.009 | H | H | $CH_2CH_3$ | Cl | Smp. 89-90° |
| 2.010 | H | H | $CH_2CH_3$ | $OCHF_2$ | Smp. 117-119° |
| 2.011 | 4-Cl | H | $CH_3$ | $CH_3$ | |
| 2.012 | H | H | $CH_3$ | Br | Smp. 170-171° |
| 2.013 | H | H | Cl | Cl | Smp. 195-196° |
| 2.014 | H | H | F | F | |
| 2.015 | H | H | Cl | $OCHF_2$ | |
| 2.016 | H | H | $OCHF_2$ | $OCHF_2$ | |
| 2.017 | H | H | $OCH_2CH_3$ | $CH_3$ | Smp. 104-105° |
| 2.018 | H | H | $SCH_3$ | $CH_3$ | Smp. 180-182° |
| 2.019 | H | H | $SCH(CH_3)_2$ | $CH_3$ | Smp. 75-77° |
| 2.020 | H | H | $SCH_3$ | Cl | Smp. 153-155° |
| 2.021 | H | H | $SC_4H_9(n)$ | Cl | |
| 2.022 | H | H | COOEt | Cl | |
| 2.023 | H | H | COOEt | $CH_3$ | |
| 2.024 | H | H | $SCH_2CH_3$ | $OCHF_2$ | |
| 2.025 | H | H | $CF_3$ | Cl | Smp. 134° |
| 2.026 | H | H | $CF_3$ | F | |
| 2.027 | H | H | $CF_3$ | $OCH_2CH_3$ | |
| 2.028 | H | H | $CF_3$ | $SCH_3$ | |
| 2.029 | H | H | $CF_3$ | $N(CH_3)_2$ | |
| 2.030 | H | H | $N(CH_3)_2$ | Cl | Smp. 175-176° |
| 2.031 | H | H | $N(CH_3)_2$ | $CH_3$ | Smp. 123-124° |
| 2.032 | H | H | $OCF_2CHF_2$ | $CH_3$ | Smp. 82-83° |
| 2.033 | H | H | $OCF_2CHF_2$ | $OCF_2CHF_2$ | |
| 2.034 | 4-$CH_3$ | H | $CH_3$ | Cl | Smp. 147-148° |

Tabelle 2 (Fortsetzung)

| Verb. Nr. | $R^1$ | $R^2$ | $R^3$ | $R^4$ | physikalische Daten |
|---|---|---|---|---|---|
| 2.035 | 4-$CH_3$ | H | $CH_3$ | $OCHF_2$ | |
| 2.036 | 4-$CH_3$ | H | $CH_3$ | F | |
| 2.037 | 4-$CH_3$ | H | $CH_3$ | $OCF_2CHF_2$ | |
| 2.038 | 4-$CH_3$ | H | $CH_3$ | $SCH_3$ | |
| 2.039 | 4-$CF_3$ | H | $CH_3$ | Cl | Smp. 135-140° |
| 2.040 | 4-$CF_3$ | H | Cl | Cl | |
| 2.041 | 4-$CF_3$ | H | $OCH_3$ | $CH_2OCH_3$ | |
| 2.042 | 4-$CF_3$ | H | $CH_3$ | H | |
| 2.043 | 4-$CF_3$ | H | H | H | |
| 2.044 | 4-Cl | H | $CH_3$ | Cl | Smp. 152-155° |
| 2.045 | 4-Cl | H | $CH_3$ | F | |
| 2.046 | 4-Cl | H | $CH_3$ | $OCHF_2$ | Smp. 118-122° |
| 2.047 | 4-Cl | H | $CH_3$ | $SCH_2CH_3$ | |
| 2.048 | 4-COOEt | H | $CH_3$ | $CH_3$ | |
| 2.049 | 4-COOEt | H | $CH_3$ | Cl | |
| 2.050 | 4-COOEt | H | $CH_3$ | $OCH_3$ | |
| 2.051 | H | 6-$CH_3$ | $CH_3$ | Cl | Smp. 149-150° |
| 2.052 | H | 6-$CH_3$ | Cl | Cl | |
| 2.053 | H | 6-$CH_3$ | Cl | $SCH_3$ | |
| 2.054 | 4-$OCH_3$ | H | $CH_3$ | Cl | Smp. 137-138° |
| 2.055 | 4-$OCH_3$ | H | $CH_3$ | $CH_3$ | |
| 2.056 | 5-$CH_3$ | H | $CH_3$ | $CH_3$ | |
| 2.057 | 5-$CH_3$ | H | $CH_3$ | $OCHF_2$ | |
| 2.058 | 5-$CH_3$ | H | $CH_3$ | Cl | |
| 2.059 | 5-$CH_3$ | H | $CH_3$ | $OCF_2CHF_2$ | |
| 2.060 | 4-Cl | 6-Cl | $CH_3$ | $CH_3$ | |
| 2.061 | 4-Cl | 6-Cl | $CH_3$ | Cl | |
| 2.062 | 4-Cl | 6-Cl | Cl | Cl | |
| 2.063 | 4-Cl | 6-Cl | $CH_3$ | F | |
| 2.064 | 4-Cl | 6-Cl | Cl | $SC_2H_5$ | |
| 2.065 | 4-$CF_3$ | 6-$NO_2$ | $CH_3$ | Cl | Smp. 143-145° |
| 2.066 | 4-$CF_3$ | 6-$NO_2$ | H | H | |
| 2.067 | 4-$CF_3$ | 6-$NO_2$ | $CH_3$ | H | |
| 2.068 | 4-$CF_3$ | 6-$NO_2$ | $CH_3$ | $OCHF_2$ | |
| 2.069 | 4-$CF_3$ | 6-$NO_2$ | $CH_3$ | $CH_2OCH_3$ | |
| 2.070 | H | H | OH | OH | Smp. 150-155° |
| 2.071 | H | H | OH | $CH_3$ | Smp. 240-242° |
| 2.072 | H | H | OH | $CH_2CH_3$ | Smp. 217-221° |
| 2.073 | 4-$CH_3$ | H | OH | $CH_3$ | Smp. 229-231° |
| 2.074 | 4-Cl | H | OH | $CH_3$ | Smp. 245-248° |
| 2.075 | H | 6-$CH_3$ | OH | $CH_3$ | |
| 2.076 | H | H | OH | $CF_3$ | Smp. 249-251° |
| 2.077 | H | 6-$CH_3$ | OH | OH | |
| 2.078 | 4-$OCH_3$ | H | OH | $CH_3$ | Smp. 225-227° |
| 2.079 | 5-$CH_3$ | H | OH | $CH_3$ | |
| 2.080 | 4-Cl | 6-Cl | OH | OH | |
| 2.082 | 4-Cl | 6-Cl | OH | $CH_3$ | |
| 2.083 | H | H | $C_3H_7$(n) | Cl | Smp. 75-76° |
| 2.084 | H | H | $C_3H_7$(n) | $OCHF_2$ | Smp. 108-112° |

Tabelle 2 (Fortsetzung)

| Verb. Nr. | $R^1$ | $R^2$ | $R^3$ | $R^4$ | physikalische Daten |
|---|---|---|---|---|---|
| 2.085 | 4-Cl | H | $CF_3$ | Cl | Smp. 102-104° |
| 2.086 | 4-Cl | H | $CF_3$ | OH | Smp. 242-245° |
| 2.087 | H | H | $CF_3$ | Br | Smp. 124-125° |
| 2.088 | 4-Cl | H | $CH_3$ | Br | Smp. 177-178° |
| 2.089 | H | H | $CH_3$ | H | Smp. 117-120° |
| 2.090 | 4-$CF_3$ | H | $CH_3$ | $CH_3$ | Smp. 136-139° |
| 2.091 | 4-$CF_3$ | H | $CH_3$ | $OCH_3$ | Smp. 120-123° |
| 2.092 | H | H | $CH_3$ | $CF_3$ | Smp. 121-123° |
| 2.093 | 5-Cl | H | $CH_3$ | OH | Smp. 225-228° |
| 2.094 | 5-Cl | H | $CH_3$ | Cl | Smp. 135-137° |
| 2.095 | H | H | $CF_3$ | $CF_3$ | Smp. 110-113° |
| 2.096 | H | 6-$CH_3$ | $CH_3$ | $CF_3$ | Smp. 118-120° |
| 2.097 | H | H | $CF_3$ | $OCH_3$ | Smp. 123-125° |
| 2.098 | H | H | $CF_3$ | $OC_4H_9(n)$ | Smp. 70-71° |
| 2.099 | H | 6-$CH_3$ | $CF_3$ | OH | |
| 2.100 | H | 6-$CH_3$ | $CF_3$ | Cl | |
| 2.101 | H | 6-$CH_3$ | $CF_3$ | $OCH_3$ | |
| 2.102 | H | 6-$CH_3$ | $CF_3$ | $OC_2H_5$ | |
| 2.103 | H | 6-$CH_3$ | $CF_3$ | $OC_3H_7(n)$ | |
| 2.104 | H | 6-$CH_3$ | $CF_3$ | $OC_4H_9(iso)$ | |
| 2.105 | H | 6-$CH_3$ | $CF_3$ | $SCH_3$ | |
| 2.106 | H | 6-$CH_3$ | $CF_3$ | $SCH(CH_3)_2$ | |
| 2.107 | H | 6-$CH_3$ | $CF_3$ | $SC_4H_9(n)$ | |
| 2.108 | H | 6-Cl | $CH_3$ | OH | |
| 2.109 | H | 6-Cl | $CH_3$ | Cl | |
| 2.110 | H | 6-Cl | $CH_3$ | $CF_3$ | |
| 2.111 | H | 6-Cl | $CF_3$ | OH | |
| 2.112 | H | 6-Cl | $CF_3$ | Cl | |
| 2.113 | H | 6-Cl | $CF_3$ | $OCH_3$ | |
| 2.114 | H | 6-Cl | $CF_3$ | $OC_2H_5$ | |
| 2.115 | H | 6-Cl | $CF_3$ | $SCH_3$ | |
| 2.116 | H | 6-Cl | $CF_3$ | $SC_2H_5$ | |

## Biologische Beispiele

### Beispiel B1: Pre-emergente Herbizid-Wirkung

Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22 - 25 °C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung.

Im einzelnen findet man bei Setaria und Stellaria bei 4 kg/ha Aufwandmengen nachstehende Werte:

| Verbindung No. | Setaria | Stellaria |
|---|---|---|
| 1.001 | 3 | 2 |
| 1.002 | 2 | 1 |
| 1.004 | 2 | 2 |
| 1.005 | 2 | 2 |
| 1.006 | 1 | 2 |
| 1.007 | 1 | 2 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die Verbindungen der Tabelle 1 gute Herbizidwirkung.

Beispiel B3 Herbizidwirkung für Wasserreis (paddy)

Die Wasserunkräuter Echinochloa crus galli und Monocharia vag. werden in Plastibechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefässe. Die verwendete Dosis entspricht einer Wirkstoffmenge von 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Appliation statt. Die Verbindungen der Tabelle 1 schädigen dabei die Unkräuter, nicht aber den Reis.

Beispiel B4: Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabelle 1 in Aufwandmengen bis zu 3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel B5: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Tabelle 1 eine Erhöhung der Anzahl und des Gewichts der Schoten im Haupttrieb.

Beispiel B6: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses, sowie teilweise eine Zunahme der Stengeldurchmesser auf.

Beispiel B7: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1 und 2 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses im Vergleich zur unbehandelten Kontrolle.

Formulierungsbeispiele

Beispiel F1: Formulierungsbeispiele für Wirkstoffe der Formel I′ (% = Gewichtsprozent)

| a) Emulsionskonzentrate | a) | b) | c) |
|---|---|---|---|
| Wirkstoff gemäss Herstellungsbeispiel 1 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gem. Herstellungsbeispiel 1 | 80 % | 10 % | 5 % |
| Aethylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Herstellungsbeispiel 1 | 5 % | 10 % |
| Kaolin | 94 % | - |
| Hochdisperse Kieselsäure | 1 % | |
| Attapulgit | - | 90 % |

Eine Wirkstofflösung wird auf den Träger aufgesprüht und das Lösungsmittel anschliessend im Vakuum abgedampft.

| d) Stäubemittel | a) | b) |
|---|---|---|
| Wirkstoff gemäss Herstellungsbeispiel 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel.

| e) Spritzpulver | a) | b) |
|---|---|---|
| Wirkstoff gemäss Herstellungbeispiel 1 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff gemäss Herstellungsbeispiel 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Patentansprüche

**1.** Verbindungen der Formel

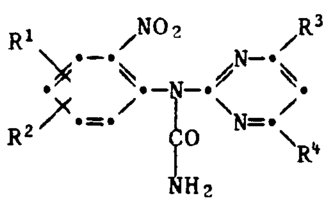

(I),

worin

$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyano,

$R^3$ und $R^4$ unabhängig voneinander Wasserstoff, Halogen, Cyano, OH, SH, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-Alkylamino, $C_1$-$C_4$-Alkoxycarbonyl oder $C_1$-$C_4$-Alkoxy-($C_1$-$C_4$)-alkyl bedeutet,

sowie deren Salze und Additionsverbindungen mit Säuren, Basen und Komplexbildnern.

**2.** Verbindungen der Formel I, gemäss Anspruch 1, worin
$R^1$ Wasserstoff, Chlor, Methyl, Trifluormethyl, Methoxy oder Ethoxycarbonyl,
$R^2$ Wasserstoff, Chlor, Methyl oder Nitro,
$R^3$ Wasserstoff, Fluor, Chlor, Methyl, Ethyl, Propyl, Trifluormethyl, Methoxy, Ethoxy, Difluromethoxy, 1,1,2,2-Tetrafluorethoxy, Dimethylamino, Ethoxycarbonyl oder $C_1$-$C_4$-Alkylthio,
$R^4$ Wasserstoff, Fluor, Chlor, Brom, Methyl, Methoxymethyl, Methoxyethyl, $C_1$-$C_4$-Alkoxy, 1,1,2,2-Tetrafluorethoxy, Dimethylamino, Difluormethoxy, Fluormethoxy, Trifluormethyl oder $C_1$-$C_4$-Alkylthio bedeutet.

**3.** Verbindungen der Formel I, gemäss Anspruch 1 oder 2, worin
$R^1$ in 5-Position des Phenylsystems gebunden ist.

**4.** Verbindungen der Formel I, gemäss einem der Ansprüche 1 bis 3, worin $R^2$ in 6-Position des Phenylsystems gebunden ist.

**5.** Verbindungen der Formel I, gemäss Anspruch 1 oder 2, worin $R^1$ und $R^2$ Wasserstoff, $R^3$ Methoxy oder Methyl und $R^4$ Chlor, Methyl, Methoxy, Trifluormethyl, Methoxymethyl oder Difluormethoxy bedeutet.

**6.** Verfahren zur Herstellung von Verbindungen der Formel I gemäss einem der Ansprüche 1 bis 5, dadurch gekennzeichnet, dass man
a) ein Anilin der Formel II mit Phosgen zu einem Carbaminchlorid der Formel III umsetzt und dieses in einer zweiten Stufe mit $NH_3$ zu

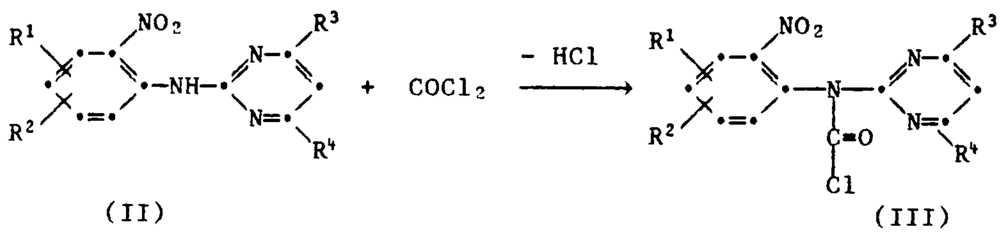


(II) (III)

$$III + NH_3 \xrightarrow{-\ HCl} I$$

einem Harnstoff der Formel I umsetzt oder
b) ein Anilin der Formel II mit Halogensulfonylisocyanat zu einem Halogensulfonylharnstoff der Formel IV umsetzt

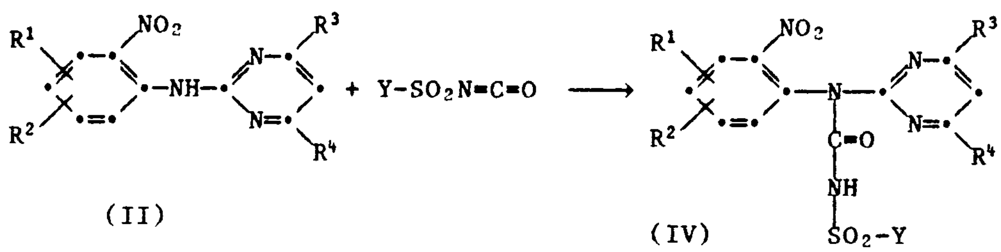


(II) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor,

steht oder

c) einen Sulfonylharnstoff der Formel V unter Einwirkung einer wässrigen Base zu einem Harnstoff der Formel I umlagert

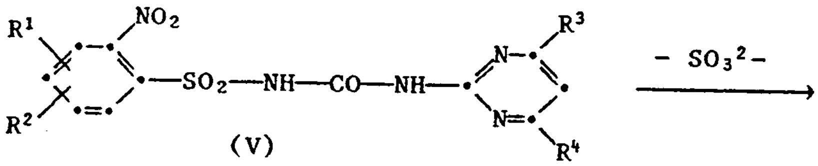


(V)

(I),

wobei als wässrige Basen vorzugsweise NaOH/Wasser oder KOH/Wasser verwendet werden.

**7.** Verbindungen der Formel

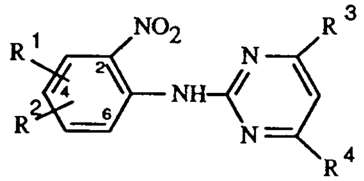

(II),

worin $R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyan, $R^3$ und $R^4$ unabhängig voneinander Wasserstoff, Halogen, Cyano, OH, SH, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-alkylamino, $C_1$-$C_4$-Alkoxycarbonyl oder $C_1$-$C_4$-Alkoxy-($C_1$-$C_4$)-alkyl bedeuten,

mit der Massgabe, dass $R^1$ nicht in 6-Stellung und $R^2$ nicht in 4-Stellung gebunden ist, wenn $R^1$ und $R^2$ für Wasserstoff, $NO_2$ oder $CF_3$ stehen, wobei nur $R^1$ oder $R^2$ für $NO_2$ stehen kann, und $R^3$ und $R^4$ Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Alkoxy($C_1$-$C_4$)alkyl ist, und $R^1$ oder $R^2$ nicht in 3-Stellung Halogen sein darf, wenn $R^2$ oder $R^1$ in 4- oder 6-Stellung Wasserstoff, $NO_2$ oder $CF_3$ ist und $R^3$ und $R^4$ Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Alkoxy($C_1$-$C_4$)alkyl ist.

**8.** Verbindungen der Formel

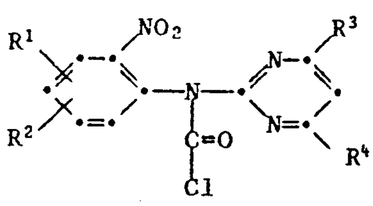

(III),

worin

$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyano,

$R^3$ und $R^4$ unabhängig voneinander Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-Alkylamino, $C_1$-$C_4$-Alkoxycarbonyl oder $C_1$-$C_4$-Alkoxy-($C_1$-$C_4$)-alkyl

bedeutet.

**9.** Harnstoffe der Formel

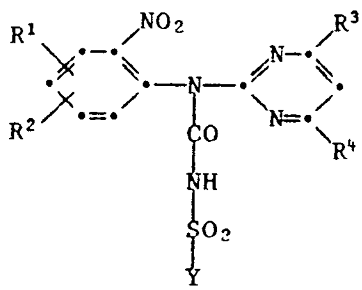

(IV),

worin
$R^1$ und $R^2$ unabhängig voneinander Wasserstoff, Halogen, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkoxy-($C_1$-$C_4$)-alkyl, Nitro oder Cyano,
$R^3$ und $R^4$ unabhängig voneinander Wasserstoff, Halogen, Cyano, $C_1$-$C_6$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, Mono-$C_1$-$C_4$-alkylamino, Di-$C_1$-$C_4$-Alkylamino, $C_1$-$C_4$-Alkoxycarbonyl oder $C_1$-$C_4$-Alkoxy-($C_1$-$C_4$)-alkyl
Y Halogen, $C_1$-$C_4$-Alkoxy oder Phenoxy
bedeutet.

**10.** Verfahren zur Herstellung von Verbindungen der Formel II gemäss Anspruch 7, dadurch gekennzeichnet, dass man
a) Guanidine der Formel VI mit 1,3-Dicarbonylverbindungen der Formel VII umsetzt,

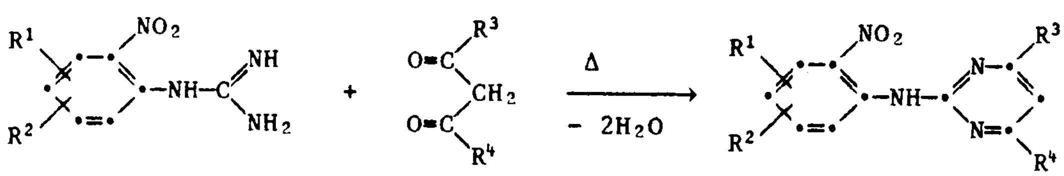


(VI) (VII) (II)

wobei die Kondensationsreaktion gewünschtenfalls in Gegenwart wasserbindender Mittel durchgeführt wird oder
b) Halogenbenzole der Formel VIII mit 2-Aminopyrimidinen der Formel IX umsetzt

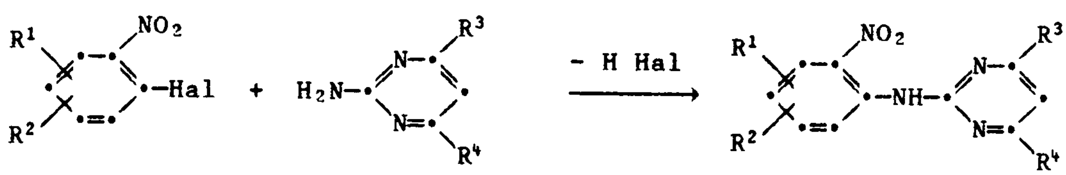


(VIII) (IX) (II)

wobei $R^1$ und $R^2$ vorzugsweise in 4 bzw. 6-Stellung des Phenylsystems (in II) gebunden sind und für eine elektronenanziehende Gruppe stehen
oder

c) einen Sulfonylharnstoff der Formel V unter Einwirkung einer wässrigen Base

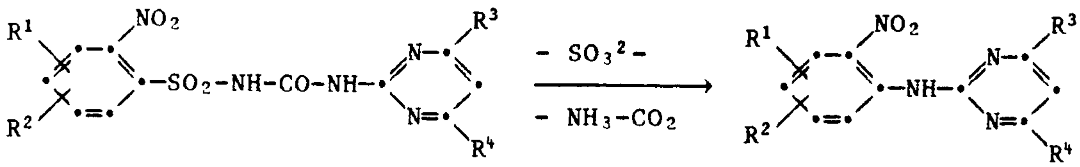


(V) (II)

bei erhöhter Temperatur zu einer Verbindung der Formel II umlagert.

**11.** Herbizides Mittel, enthaltend als Wirksubstanz eine Verbindung der Formel I, gemäss einem der Ansprüche 1 bis 5, neben weiteren Hilfs- und/oder Trägerstoffen.

**12.** Pflanzenwuchsregulatorisches Mittel, enthaltend als Wirksubstanz eine Verbindung der Formel I, gemäss einem der Anpsrüche 1 bis 5, neben weiteren Hilfs- und/oder Tägerstoffen.

**13.** Verfahren zur Bekämpfung unerwünschten Pflanzenwuchses, dadurch gekennzeichnet, dass man eine herbizid wirksame Menge einer Wirksubstanz der Formel I gemäss einem der Ansprüche 1 bis 5 auf die zu bekämpfende Pflanze oder deren Lebensraum einwirken lässt.

**14.** Verfahren zur Beeinflussung des Pflanzenwuches, dadurch gekennzeichnet, dass man eine pflanzenwachstumsregulatorisch wirksame Menge einer Wirksubstanz der Formel I gemäss einem der Ansprüche 1 bis 5 auf die Pflanze oder deren Lebensraum einwirken lässt.

## Claims

**1.** A compound of formula

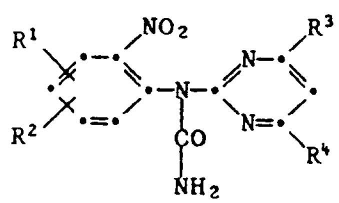

(I)

in which
$R^1$ and $R^2$ are each, independently of the other, hydrogen, halogen, $C_1$-$C_6$alkyl, $C_1$-$C_4$haloalkyl, $C_1$-$C_4$alkoxy, $C_1$-$C_4$-haloalkoxy, $C_1$-$C_4$alkoxycarbonyl, $C_1$-$C_4$alkoxy-($C_1$-$C_4$)-alkyl, nitro or cyano, and
$R^3$ and $R^4$ are each, independently of the other, hydrogen, halogen, cyano, OH, SH, $C_1$-$C_6$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, amino, mono-$C_1$-$C_4$-alkylamino, di-$C_1$-$C_4$alkylamino, $C_1$-$C_4$alkoxycarbonyl or $C_1$-$C_4$alkoxy-($C_1$-$C_4$)alkyl, or a salt or an addition compound thereof with an acid, base or complex former.

**2.** A compound of formula I according to claim 1 in which
$R^1$ is hydrogen, chlorine, methyl, trifluoromethyl, methoxy or ethoxycarbonyl,
$R^2$ is hydrogen, chlorine, methyl or nitro,
$R^3$ is hydrogen, fluorine, chlorine, methyl, ethyl, propyl, trifluoromethyl, methoxy, ethoxy, difluoromethoxy, 1,1,2,2-tetrafluoroethoxy, dimethylamino, ethoxycarbonyl or $C_1$-$C_4$alkylthio, and
$R^4$ is hydrogen, fluorine, chlorine, bromine, methyl, methoxymethyl, methoxyethyl, $C_1$-$C_4$alkoxy, 1,1,2,2-tetrafluoroethoxy, dimethylamino, difluoromethoxy, fluoromethoxy, trifluoromethyl or $C_1$-$C_4$alkylthio.

3. A compound of formula I according to claim 1 or claim 2 in which $R^1$ is bonded in the 5-position of the phenyl system.

4. A compound of formula I according to any one of claims 1 to 3 in which $R^2$ is bonded in the 6-position of the phenyl system.

5. A compound of formula I according to claim 1 or claim 2 in which $R^1$ and $R^2$ are hydrogen, $R^3$ is methoxy or methyl and $R^4$ is chlorine, methyl, methoxy, trifluoromethyl, methoxymethyl or difluoromethoxy.

6. A process for the preparation of a compound of formula I according to any one of claims 1 to 5, which comprises
   a) reacting an aniline of formula II with phosgene to form a carbamine chloride of formula III and, in a second stage, reacting that with $NH_3$ to form a urea of formula I

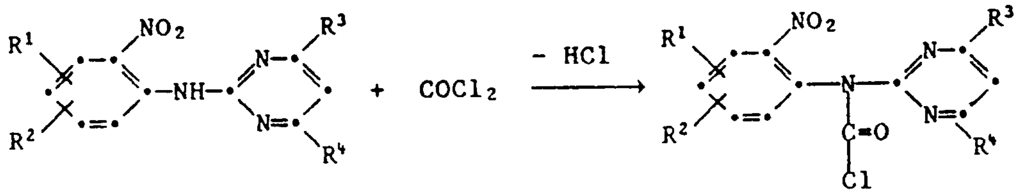


(II) (III)

$$III + NH_3 \xrightarrow{-\ HCl} I$$

or
   b) reacting an aniline of formula II with halosulphonyl isocyanate to form a halosulphonylurea of formula IV

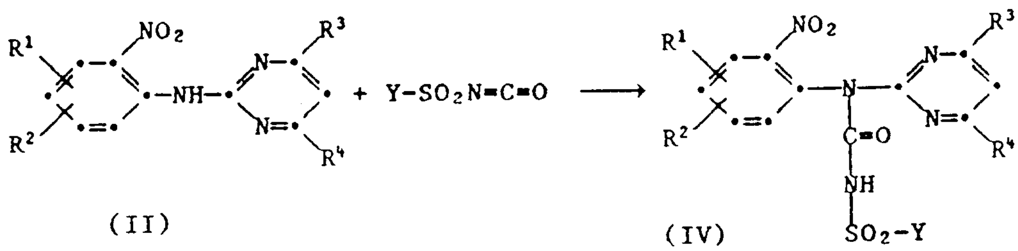


(II) (IV)

$$IV + 2H_2O \xrightarrow[-\ SO_4H_2]{-\ HY} I$$

and hydrolysing that, in a second stage or directly, to form a compound of formula I, Y in the above formulae representing a group that can be removed under the reaction conditions, such as halogen, preferably chlorine, or
   c) rearranging a sulphonylurea of formula V under the action of an aqueous base to form a urea of formula I

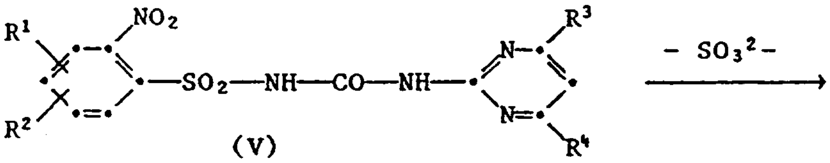


(V) (I)

the aqueous base used preferably being NaOH/water or KOH/water.

**7.** A compound of formula

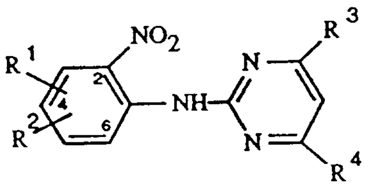

(II)

in which
$R^1$ and $R^2$ are each, independently of the other, hydrogen, halogen, $C_1$-$C_6$alkyl, $C_1$-$C_4$haloalkyl, $C_1$-$C_4$alkoxy, $C_1$-$C_4$-haloalkoxy, $C_1$-$C_4$alkoxycarbonyl, $C_1$-$C_4$alkoxy-($C_1$-$C_4$)-alkyl, nitro or cyano, and
$R^3$ and $R^4$ are each, independently of the other, hydrogen, halogen, cyano, OH, SH, $C_1$-$C_6$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, amino, mono-$C_1$-$C_4$-alkylamino, di-$C_1$-$C_4$alkylamino, $C_1$-$C_4$alkoxycarbonyl or $C_1$-$C_4$alkoxy-($C_1$-$C_4$)alkyl, with the proviso that when $R^1$ and $R^2$ are hydrogen, $NO_2$ or $CF_3$ $R^1$ is not bonded in the 6-position and $R^2$ is not bonded in the 4-position, wherein only $R^1$ or $R^2$ can be $NO_2$ and $R^3$ and $R^4$ are hydrogen, halogen, cyano, $C_1$-$C_6$alkyl, $C_1$-$C_4$haloalkyl, $C_1$-$C_4$alkoxy, $C_1$-$C_4$haloalkoxy, $C_1$-$C_4$alkylthio or $C_1$-$C_4$alkoxy($C_1$-$C_4$)alkyl, and $R^1$ or $R^2$ may not be halogen in the 3-position when $R^2$ or $R^1$ in the 4- or 6-position is hydrogen, $NO_2$ or $CF_3$ and $R^3$ and $R^4$ are hydrogen, halogen, cyano, $C_1$-$C_6$alkyl, $C_1$-$C_4$haloalkyl, $C_1$-$C_4$alkoxy, $C_1$-$C_4$haloalkoxy, $C_1$-$C_4$alkylthio or
$C_1$-$C_4$alkoxy($C_1$-$C_4$)alkyl.

**8.** A compound of formula

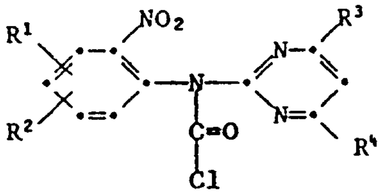

(III)

in which
$R^1$ and $R^2$ are each, independently of the other, hydrogen, halogen, $C_1$-$C_6$alkyl, $C_1$-$C_4$haloalkyl, $C_1$-$C_4$alkoxy, $C_1$-$C_4$-haloalkoxy, $C_1$-$C_4$alkoxycarbonyl, $C_1$-$C_4$alkoxy-($C_1$-$C_4$)-alkyl, nitro or cyano, and
$R^3$ and $R^4$ are each, independently of the other, hydrogen, halogen, cyano, $C_1$-$C_6$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, amino, mono-$C_1$-$C_4$alkylamino, di-$C_1$-$C_4$alkylamino, $C_1$-$C_4$alkoxycarbonyl or $C_1$-$C_4$alkoxy-($C_1$-$C_4$)alkyl.

**9.** A urea of formula

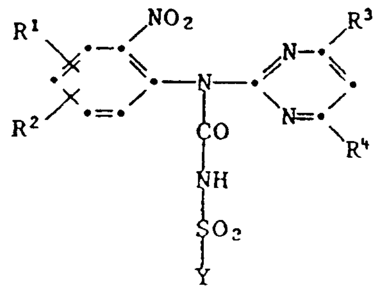

(IV)

in which
$R^1$ and $R^2$ are each, independently of the other, hydrogen, halogen, $C_1$-$C_6$alkyl, $C_1$-$C_4$haloalkyl, $C_1$-$C_4$alkoxy, $C_1$-$C_4$-haloalkoxy, $C_1$-$C_4$alkoxycarbonyl, $C_1$-$C_4$alkoxy-($C_1$-$C_4$)-alkyl, nitro or cyano, and
$R^3$ and $R^4$ are each, independently of the other, hydrogen, halogen, cyano, $C_1$-$C_6$alkyl, $C_1$-$C_4$haloalkyl, $C_1$-$C_4$alkoxy, $C_1$-$C_4$haloalkoxy, $C_1$-$C_4$alkylthio, $C_1$-$C_4$haloalkylthio, amino, mono-$C_1$-$C_4$alkylamino, di-$C_1$-$C_4$alkylamino, $C_1$-$C_4$-alkoxycarbonyl or $C_1$-$C_4$alkoxy-($C_1$-$C_4$)alkyl, and
Y is halogen, $C_1$-$C_4$alkoxy or phenoxy.

**10.** A process for the preparation of a compound of formula II according to claim 7, which comprises
a) reacting a guanidine of formula VI with a 1,3-dicarbonyl compound of formula VII

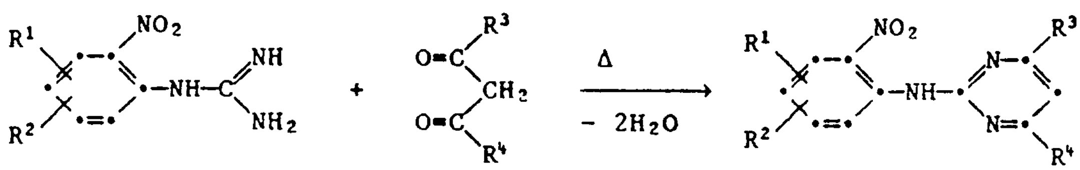


(VI) (VII) (II)

the condensation reaction if desired being carried out in the presence of a water-binding agent or
b) reacting a halobenzene of formula VIII with a 2-aminopyrimidine of formula IX

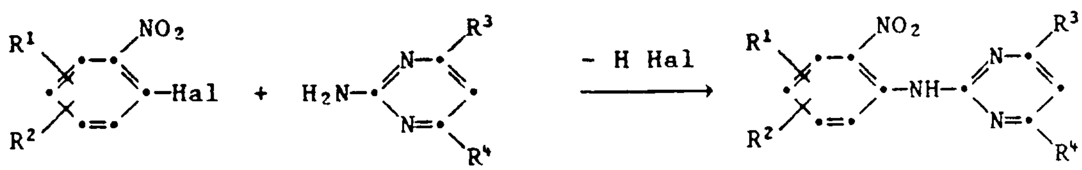


(VIII) (IX) (II)

in which $R^1$ and $R^2$ are bonded preferably in the 4- and 6-positions, respectively, of the phenyl system (in II) and represent an electron-attracting group, or
c) rearranging a sulphonylurea of formula V at elevated temperature under the action of an aqueous base to form a compound of formula II

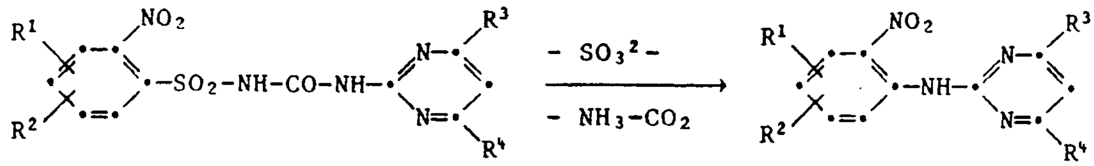


(V) (II)

**11.** A herbicidal composition comprising as active ingredient a compound of formula I according to any one of claims 1 to 5 together with other adjuvants and/or carriers.

**12.** A plant growth-regulating composition comprising as active ingredient a compound of formula I according to any one of claims 1 to 5 together with other adjuvants and/or carriers.

**13.** A method of controlling undesired plant growth, which comprises allowing a herbicidally effective amount of an active ingredient of formula I according to any one of claims 1 to 5 to act on the plant to be controlled or the locus thereof.

**14.** A method of influencing plant growth, which comprises allowing an amount of an active ingredient of the formula I according to any one of claims 1 to 5 that is effective for plant growth regulation to act on the plant or the locus thereof.

## Revendications

**1.** Composés de formule générale :

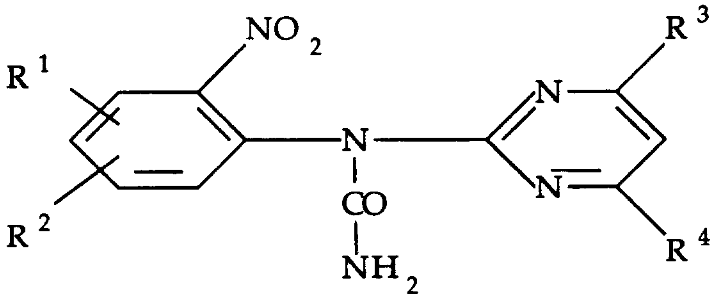

dans laquelle :

$R^1$ et $R^2$ représentent chacun, indépendamment l'un de l'autre, l'hydrogène, un halogène, un groupe alkyle en $C_1$-$C_6$, halogénoalkyle en $C_1$-$C_4$, alcoxy en $C_1$-$C_4$, halogénoalcoxy en $C_1$-$C_4$, (alcoxy en $C_1$-$C_4$)-carbonyle, (alcoxy en $C_1$-$C_4$)-alkyle en $C_1$-$C_4$, nitro ou cyano,

$R^3$ et $R^4$ représentent chacun, indépendamment l'un de l'autre, l'hydrogène, un halogène, un groupe cyano, OH, SH, alkyle en $C_1$-$C_6$, halogénoalkyle en $C_1$-$C_4$, alcoxy en $C_1$-$C_4$, halogénoalcoxy en $C_1$-$C_4$, alkylthio en $C_1$-$C_4$, halogénoalklthio en $C_1$-$C_4$, amino, mono-(alkyle en $C_1$-$C_4$)-amino, di-(alkyle en $C_1$-$C_4$)-amino, (alcoxy en $C_1$-$C_4$)-carbonyle ou (alcoxy en $C_1$-$C_4$)-alkyle en $C_1$-$C_4$,

leurs sels et leurs composés d'addition avec des acides, des bases et des agents complexants.

**2.** Composés de formule I selon la revendication 1, dans laquelle :

$R^1$ représente l'hydrogène, le chlore, un groupe méthyle, trifluorométhyle, méthoxy ou éthoxycarbonyle,

$R^2$ représente l'hydrogène, le chlore, un groupe méthyle ou nitro,

$R^3$ représente l'hydrogène, le fluor, le chlore, un groupe méthyle, éthyle, propyle, trifluorométhyle, méthoxy, éthoxy, difluorométhoxy, 1,1,2,2-tétra-fluoréthoxy, diméthylamino, éthorycarbonyle ou alkylthio en $C_1$-$C_4$,

$R^4$ représente l'hydrogène, le fluor, le chlore, le brome, un groupe méthyle, méthoxyméthyle,

méthoxyéthyle, alcoxy en $C_1$-$C_4$, 1,1,2,2-tétrafluoréthoxy, diméthylamino, difluorométhoxy, fluorméthoxy, trifluorométhyle ou alkylthio en $C_1$-$C_4$.

**3.** Composés de formule I selon la revendication 1 ou 2, dans laquelle $R^1$ est relié en position 5 du système phényle.

**4.** Composés de formule I selon l'une des revendications 1 à 3, dans laquelle $R^2$ est relié en position 6 du système phényle.

**5.** Composés de formule I selon la revendication 1 ou 2, dans laquelle $R^1$ et $R^2$ représentent l'hydrogène, $R^3$ un groupe méthoxy ou méthyle et $R^4$ le chlore, un groupe méthyle, méthoxy, trifluorométhyle, méthoxyméthyle ou difluorométhoxy.

**6.** Procédé de préparation des composés de formule I selon l'une des revendications 1 à 5, caractérisé en ce que :

a) on fait réagir une aniline de formule II avec le phosgène, ce qui donne un chlorure d'acide carbamique de formule III qu'on convertit dans un deuxième stade, par réaction avec $NH_3$, en une urée de formule I :

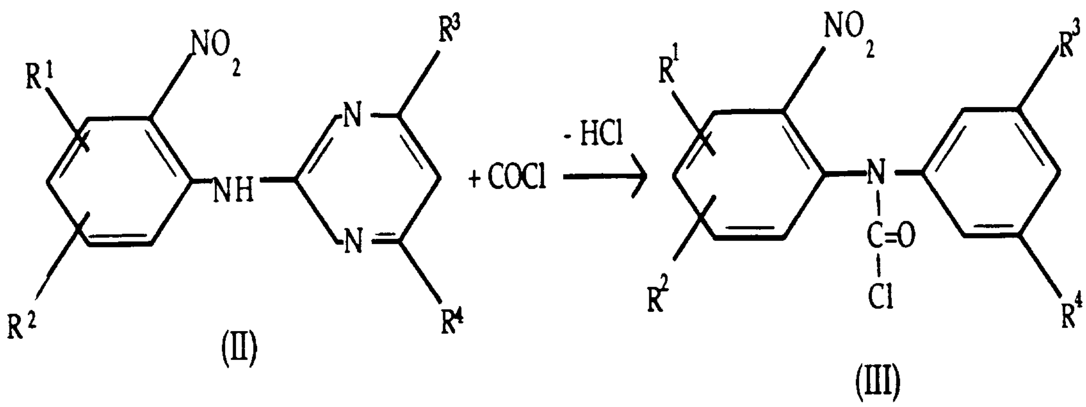

(II)
(III)

$$III + NH_3 \xrightarrow{-HCl} I$$

ou bien

b) on fait réagir une aniline de formule II avec un isocyanate d'halogénosulfonyle, ce qui donne une halogénosulfonylurée de formule IV :

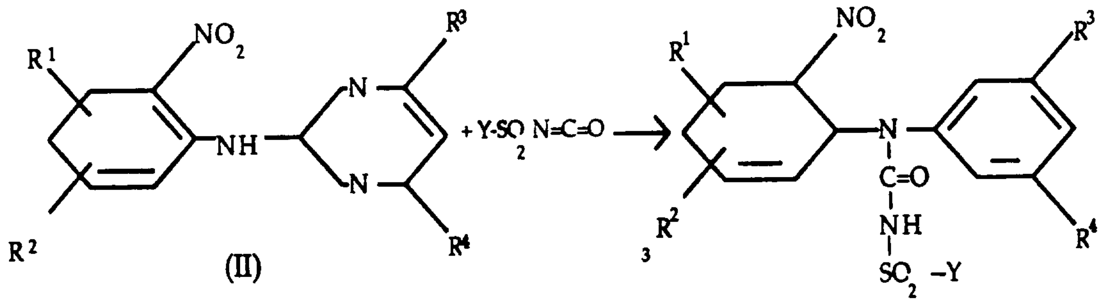


(II)

(IV)

$$IV + 2H_2O \xrightarrow[-SO_4H_2]{-HY} I$$

qu'on hydrolyse dans un deuxième stade ou directement en un composé de formule I, Y représentant un substituant éliminable dans les conditions de la réaction, par exemple un halogène, de préférence le chlore, ou bien

c) on transpose une sulfonylurée de formule V sous l'action d'une base aqueuse en une urée de formule I :

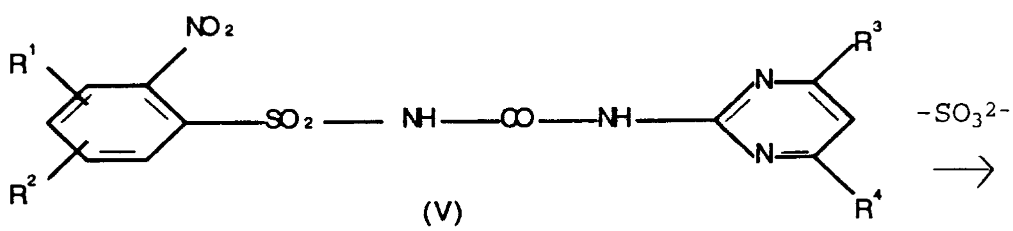


(V)

(I)

la base aqueuse consistant de préférence en lessive de soude ou lessive de potasse.

**7.** Composés de formule :

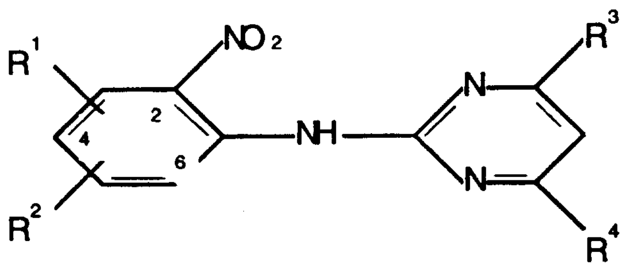

(II)

dans laquelle :

$R^1$ et $R^2$ représentent chacun, indépendamment l'un de l'autre, l'hydrogène, un halogène, un groupe

alkyle en $C_1$-$C_6$, halogénoalkyle en $C_1$-$C_4$, alcoxy en $C_1$-$C_4$, halogénoalcoxy en $C_1$-$C_4$, (alcoxy en $C_1$-$C_4$)-carbonyle, (alcoxy en $C_1$-$C_4$)-alkyle en $C_1$-$C_4$, nitro ou cyano,

$R^3$ et $R^4$ représentent chacun, indépendamment l'un de l'autre, l'hydrogène, un halogène, un groupe cyano, OH, SH, alkyle en $C_1$-$C_6$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, amino, mono-(alkyle en $C_1$-$C_4$)-amino, di-(alkyle en $C_1$-$C_4$)-amino, (alcoxy en $C_1$-$C_4$)-carbonyle ou (alcoxy en $C_1$-$C_4$)-alkyle en $C_1$-$C_4$,

sous réserve que $R^1$ ne peut être relié en position 6 et $R^2$ ne peut être relié en position 4 lorsque $R^1$ et $R^2$ représentent l'hydrogène, $NO_2$ ou $CF_3$, un seul des symboles $R^1$ ou $R^2$ pouvant représenter $NO_2$, et $R^3$ et $R^4$ représentent l'hydrogène, des halogènes, des groupes cyano, alkyle en $C_1$-$C_6$, halogénoalkyle en $C_1$-$C_4$, alcoxy en $C_1$-$C_4$, halogénoalcoxy en $C_1$-$C_4$, alkylthio en $C_1$-$C_4$, ou (alcoxy en $C_1$-$C_4$)-alkyle en $C_1$-$C_4$, et $R^1$ ou $R^2$ ne peut représenter un halogène en position 3 lorsque $R^2$ ou $R^1$ représente en position 4 ou 6 l'hydrogène, $NO_2$ ou $CF_3$ et $R^3$ et $R^4$ représentent l'hydrogène, des halogènes, des groupes cyano, alkyle en $C_1$-$C_6$, halogénoalkyle en $C_1$-$C_4$, alcoxy en $C_1$-$C_4$, halogénoalcoxy en $C_1$-$C_4$, alkylthio en $C_1$-$C_4$ ou (alcoxy en $C_1$-$C_4$)-alkyle en $C_1$-$C_4$.

**8.** Composés de formule :

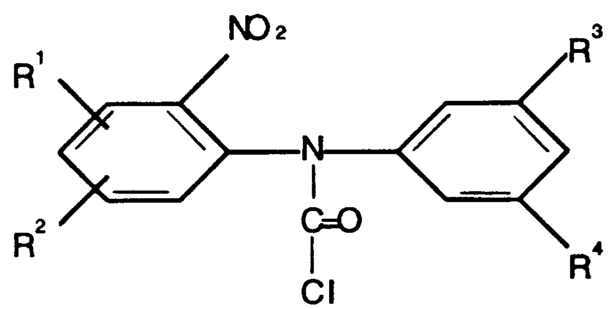

(III)

dans laquelle :

$R^1$ et $R^2$ représentent chacun, indépendamment l'un de l'autre, l'hydrogène, un halogène, un groupe alkyle en $C_1$-$C_6$, halogénoalkyle en $C_1$-$C_4$, alcoxy en $C_1$-$C_4$, halogénoalcoxy en $C_1$-$C_4$, (alcoxy en $C_1$-$C_4$)-carbonyle, (alcoxy en $C_1$-$C_4$)-alkyle en $C_1$-$C_4$, nitro ou cyano,

$R^3$ et $R^4$ représentent chacun, indépendamment l'un de l'autre, l'hydrogène, un halogène, un groupe cyano, OH, SH, alkyle en $C_1$-$C_6$, halogénoalkyle en $C_1$-$C_4$, alcoxy en $C_1$-$C_4$, halogénoalcoxy en $C_1$-C4, alkylthio en $C_1$-$C_4$, halogénoalkylthio en $C_1$-$C_4$, amino, mono-(alkyle en $C_1$-$C_4$)-amino, di-(alkyle en $C_1$-$C_4$)-amino, (alcoxy en $C_1$-$C_4$)-carbonyle ou (alcoxy en $C_1$-$C_4$)-alkyle en $C_1$-$C_4$.

**9.** Urées de formule :

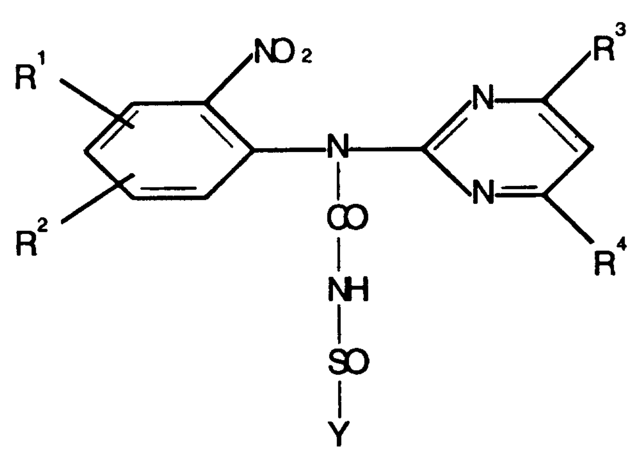

(IV)

dans laquelle :

$R^1$ et $R^2$ représentent chacun, indépendamment l'un de l'autre, l'hydrogène, un halogène, un groupe alkyle en $C_1$-$C_6$, halogénoalkyle en $C_1$-$C_4$, alcoxy en $C_1$-$C_4$, halogénoalcoxy en $C_1$-$C_4$, (alcoxy en $C_1$-$C_4$)-carbonyle, (alcoxy en $C_1$-$C_4$)-alkyle en $C_1$-$C_4$, nitro ou cyano,

$R^3$ et $R^4$ représentent chacun, indépendamment l'un de l'autre, l'hydrogène, un halogène, un groupe cyano, OH, SH, alkyle en $C_1$-$C_6$, halogénoalkyle en $C_1$-$C_4$, alcoxy en $C_1$-$C_4$, halogénoalcoxy en $C_1$-C4, alkylthio en $C_1$-$C_4$, halogénoalkylthio en $C_1$-$C_4$, amino, mono-(alkyle en $C_1$-$C_4$)-amino, di-(alkyle en $C_1$-$C_4$)-amino, (alcoxy en $C_1$-$C_4$)-carbonyle ou (alcoxy en $C_1$-$C_4$)-alkyle en $C_1$-$C_4$,

Y représente un halogène, un groupe alcoxy en $C_1$-$C_4$ ou phénoxy.

**10.** Procédé de préparation des composés de formule II de la revendication 7, caractérisé en ce que :

a) on fait réagir des guanidines de formule VI avec des dérivés 1,3-dicarbonylés de formule VII :

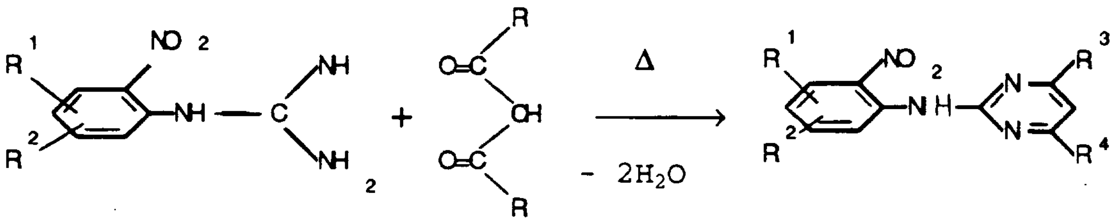


la réaction de condensation étant effectuée si on le désire en présence d'agents déshydratants, ou bien

b) on fait réagir des halogénobenzènes de formule VIII avec des 2-aminopyrimidines de formule IX :

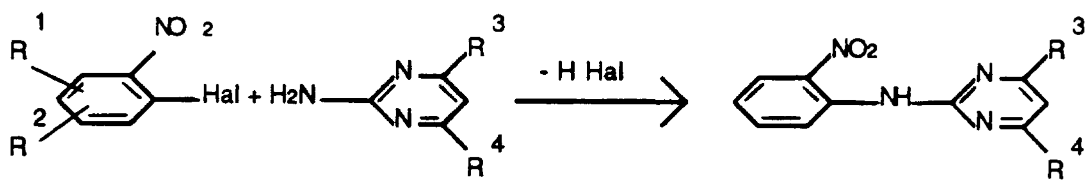


(VIII) (IX) (II)

$R^1$ et $R^2$ étant reliés de préférence dans les positions respectives 4 et 6 du système phényle (de II) et représentant chacun un groupe accepteur d'électrons, ou bien

c) on transpose une sulfonylurée de formule V sous l'action d'une base aqueuse :

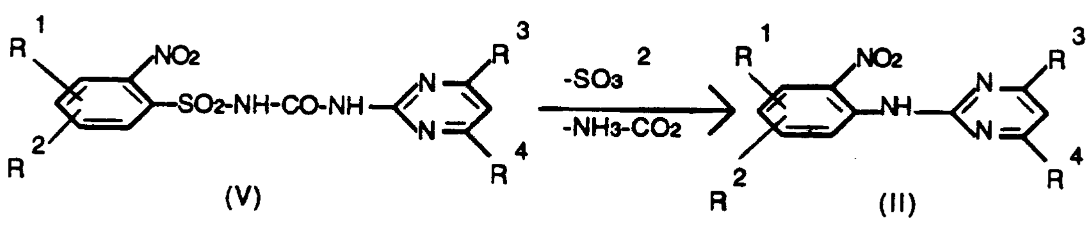


(V) (II)

à température élevée, en un composé de formule II.

**11.** Produit herbicide contenant en tant que substance active un composé de formule I selon l'une des revendications 1 à 5, avec des produits auxiliaires et/ou véhicules.

**12.** Produit régulateur de la croissance des végétaux contenant en tant que substance active un composé de formule I selon l'une des revendications 1 à 5, avec des produits auxiliaires et/ou des véhicules.13.- Procédé pour combattre les croissances de végétaux indésirables, caractérisé en ce que l'on fait agir une quantité herbicide efficace d'une substance active de formule I selon l'une des revendications 1 à 5 sur la plante à combattre ou son habitat.

**13.** Procédé pour combattre les croissances de végétaux indésirables, caractérisé en ce que l'on fait agir une quantité herbicide efficace d'une substance active de formule I selon l'une des revendications 1 à 5 sur la plante à combattre ou son habitat.

**14.** Procédé pour agir sur la croissance des végétaux, caractérisé en ce que l'on fait agir une quantité efficace en tant que régulateur de croissance dès végétaux, d'une substance active de formule I selon les revendications 1 à 5 sur la plante ou son habitat.